# EUROPEAN PATENT APPLICATION

(11) **EP 1 679 075 A1**
(43) Date of publication of application: **12.07.2006**
(21) Application number: 04793199.3
(22) Date of filing: 29.10.2004
(51) Int. Cl.: A61K 31/48, A61K 9/70, A61K 47/32, A61P 25/16

(54) **TRANSDERMAL PREPARATIONS AND METHOD FOR RELIEVING SIDE EFFECTS IN PERGOLIDE THERAPY**

(30) Priority: 31.10.2003 JP 2003373601
(71) Applicant: HISAMITSU PHARMACEUTICAL CO. INC., Tosu-shi, Saga-ken 841-0017 (JP)
(72) Inventor: TOSHIMITSU, A.; c/o HISAMITSU PHARMA CO., INC., Tsukuba-shi, Ibaraki 3050856 (JP); AIDA, K.; c/o HISAMITSU PHARMA CO., INC., Tsukuba-shi, Ibaraki 3050856 (JP); TERAHARA, T.; c/o HISAMITSU PHARMA CO., INC., Tsukuba-shi, Ibaraki 3050856 (JP); HIGO, N.; c/o HISAMITSU PHARMA CO., INC., Tsukuba-shi, Ibaraki 3050856 (JP)
(74) Representative: Schnappauf, Georg
(86) International application number: PCT/JP2004/016091
(87) International publication number: WO 2005/041967

(57) **Abstract**

It is intended to provide pergolide-containing transdermal preparations having reducreduced side effects and exerting sufficient therapeutic effects. Namely, a pergolide-containing transdermal preparation which is capable of achieving a plasma AUC ratio of pergolide or the like to at least one pergolide metabolite of 1:0.5 to 1:5; and/or transdermal preparation containing pergolide and/or a pharmaceutically acceptable salt thereof which is capable of achieving a ratio (A/B) of the maximum plasma level (A) of pergolide and/or a pharmaceutically acceptable salt thereof to the plasma level (B) thereof in the next administration of less than 2.

## Description

### [Technical Field]

The invention relates to a transdermal preparation which contains pergolide known as an anti-parkinsonism agent and/or a parmaceutically acceptable salt thereof (hereinafter described as pergolide or the like), is remarkably excellent in a skin permeability and low in skin irritation. More particularly, the invention relates to a transdermal pergolide preparation for a Parkinson's disease therapy, in which side effects are reduced by not only reducing a plasma level change of pergolide itself but suppressing formation of a metabolite of pergolide or the like (hereinafter described as a pergolide metabolite), and a method of reducing side effects in a pergolide therapy.

### [Background Art]

In a drug therapy for Parkinson's disease, in order to compensate a reduced dopamine the administration of levodopa which is its precursor is generally effective, however, the effect is reduced in accordance to a long term administration, so that coadministration with a drug which is different in the action mechanism is carried out. Pergolide mesylate is one of dopamine agonists, and is widely used in a clinical treatment because its coadministration with levodopa not only can give a dose reduction of dopamine but can reduce its side effects.

However, the pergolide mesylate preparation, which can be used at present, is a tablet, and can not avoid metabolism/degradation in the liver, whereby the administered drug is not utilized effectively and has a problem that gastric side effects, for example, represented by nausea, vomiting, stomach discomfort and the like tends to appear. In addition, since generally in a parkinsonism patient, a gastric function is lowered, there is such a problem that the bioavailability of pergolide by oral administration does not easily become constant. In particular, in order to avoid side effects accompanied by an abrupt rise of a plasma level of pergolide or the like and/or a metabolite thereof which results from change of the above bioavailability and effect of metabolism, in case of orally administering pergolide mesylate it gradual increase of one day dose is necessary for carrying out administration . Namely, 50 µg/day is administered till the second day after administration, and then every two or three days 50 µg/day is added as one day dosage. It is compelled to adopt an extremely tedious dose-setting that at the end of the first week or the second week, 150µg and 600µg are administered respectively as one day dose, and at the third week one day dose starts from 750µg, and then a dose addition is made considering the efficacy and safety and a maintenance dose (as a standard 750-1250 µg/day) is determined (Non-patent document 1). In order to dissolve the above tedious dose-setting and instability of the effect accompanying this, in recent years several transdermal preparations containing pergolide or the like as a preparation which substitutes for an oral preparation have been proposed.

For example, in patent document 1 a composition for a transdermal administration by combination of a pharmaceutically acceptable salt of pergolide with a permeation enhancer is proposed. In addition, as a device to increase a skin permeability, in patent document 2 a matrix composition containing a layered pergolide and in patent document 3 a multiple layer transdermal preparation containing water are disclosed. Further, in patent documents 4 and 5, in a plaster of pergolide a method to strengthen the action by blend of an additional pharmaceutical ingredient and a pergolide-containing adhesive patch preparation which is low in skin irritation and excellent in physical stability are disclosed. In addition, in patent document 6 an adhesive patch to give an administration effect of a drug including pergolide in sufficiently high standard and stable state is disclosed.
However, in preparations described in any of the above documents, reducing of the above side effects due to pergolide or the like is disregarded from the objective, whereby said reducing is not described.

In patent document 7, a transdermal preparation, which increases skin permeability of a drug having ergoline skeleton like pergolide and also seeks reducing of side effects of the drug, is disclosed. However, the reducing method of side effects in said document mainly depends on a change of a dosage form from an oral form to a transdermal form.
In patent document 8, a method and a preparation to reduce side effects of oxybutynin is disclosed. However, said document fails to provide any mention on a transdermal preparation containing pergolede as well as a therapeutic drug for Parkinson's disease: The document even fails to provide any specific information on reducing of side effects due to pergolide or the like.
Namely, although it has been tired to avoid influence due to metabolism by adopting a transdermal dosage-form and to sufficiently exert the drug effect of pergolide, a transdermal preparation to simultaneously suppress the side effects is yet to be obtained. Therefore, the development of a transdermal preparation which can be used in pergolide therapy is desired.

Patent document 1: JP, 11-507361 A
Patent document 2: JP, 2002-515424 A
Patent document 3: WO02/078602
Patent document 4: JP, 2000-514053 A
Patent document 5: WO02/38139
Patent document 6: WO02/69942
Patent document 7: WO03/13611
Patent document 7: U.S., A, 2002/0147236
Non-patent document 8: DRUGS IN JAPAN, 26th Edition, edited by JAPAN PHARMACEUTICAL INFORMATION CENTER, 2003, p.2039

### [Disclosure of the Invention]

### [Problem to Be Solved by the Invention]

Thus, the object of the invention is to provide a transdermal preparation containing pergolide or the like which has not the above problems, that is, reduces side effects and exerts a sufficient therapeutic effect.

### [Means for Solving Problem]

During extensive research to solve the above problems in the prior arts described above, the inventors surprisingly found out that the above problems can be solved by suppressing pergolide metabolite formation or a peak appearance of the plasma level of a pergolide metabolite, and completed the invention as a result of further investigation.

Namely, the invention relates to a transdermal preparation containing pergolide or the like, which is capable of achieving a plasma AUC ratio of pergolide or the like to at least one pergolide metabolite of 1:0.5 to 1:5.
In addition, the invention relates to the above transdermal preparation, wherein the plasma AUC ratio of pergolide or the like to at least one pergolide metabolite of 1:0.5 to 1:3.5.
Further, the invention relates to the above transdermal preparation, wherein the plasma AUC ratio of pergolide or the like to at least one pergolide metabolite is 1:0.5 to 1:2.
In addition, the invention relates to the above transdermal preparation, wherein the pergolide metabolite is one or more kinds comprising pergolide sulfoxide, pergolide sulfone, despropyl pergolide or despropyl pergolide sulfoxide.
Further, the invention relates to the above transdermal preparation, wherein the pergolide metabolite is pergolide sulfoxide.

In addition, the invention relates to the above transdermal preparation, wherein the pharmaceutically acceptable salt is one or more kinds comprising hydrochloride, sulfate, mesylate, citrate, fumarate, tartarate, maleate or acetate.
Further, the invention relates to the above transdermal preparation, wherein the pharmaceutically acceptable salt is mesylate.
In addition, the invention relates to the above transdermal preparation, wherein the ratio (A/B) of the maximum plasma level (A) of pergolide and/or the pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2.
Further, the invention relates to the above transdermal preparation, wherein (meth)acrylic acid copolymer is contained in an adhesive layer.
Furthermore, the invention relates to the above transdermal preparation, wherein the acrylic polymer except (meth) acrylic acid copolymer is further contained in an adhesive layer.
Further, the invention relates to the above transdermal preparation containing pergolide and/or the pharmaceutically acceptable salt thereof, wherein the ratio (A/B) of the maximum plasma level (A) of pergolide and/or the pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2.
Furthermore, the invention relates to any one of the transdermal preparations described above, which is an adhesive patch.

In a transdermal preparation of the invention, by achieving a plasma AUC ratio of pergolide or the like to at least one pergolide metabolite of 1:0.5 to 1:5, the amount of a pergolide metabolite produced, which is causative for side effects, can be reduced, so that the side effects in pergolide therapy can remarkably be reduced.

On the other hand, in an oral administration of pergolide a peak of the plasma level of pergolide or the like and/or pergolide sulfoxide, which is a main metabolite thereof, in the first administration is significantly higher compared with that in the next administration and appears in an earlier stage. Namely, a ratio (A/B) the maximum plasma level (A) in the first administration to the plasma level (B) in the next administration is large, so that, as the result, a risk of side effects in the first administration is not only high but an effective blood level in the next administration becomes difficult to be achieved, which may cause insufficient therapeutic effects. Considering the information, the transdermal preparation of the invention suppresses the peak appearance of pergolide or the like and/or pergolide sulfoxide,thus reducing the side effects.
Namely, by using a pergolide-containing transdermal preparation of the invention, it is possible to have pergolide and/or a pharmaceutically acceptable salt thereof absorbed effectively in circulating blood via the skin, so that the preparation can simply be administered. In addition, by the pergolide-containing transdermal preparation of the invention, it is possible to reduce the side effects by suppressing a metabolite formation and/or of the peak appearance of pergolide or the like and/or pergolide sulfoxide, so that the pergolide-containing transdermal preparation of the invention is useful as a preparation for external use for a transdermal application of pergolide in a parkinsonism therapy.

### [Effect of the Invention]

The invention is a pergolide-containing transdermal preparation,which is capable of achieving a plasma AUC ratio of pergolide or the like to at least one pergolide metabolite of 1:0.5 to 1:5 and reducing side effects in a pergolide therapy. Therefore, by a transdermal preparation of the invention, side effects in the pergolide therapy can be reduced.
In addition,the transdermal preparations of the invention, which enable a plasma AUC ratio of pergolide or the like to at least one pergolide metabolite of 1:0.5 to 1:3.5, more preferably 1:0.5 to 1:2, enable reduction of the formation of at least one pergolide metabolite, so that more reamarkable reduction of the side effects in the pergolide therapy being achieved.

In addition, the transdermal preparations of the invention, in which at least one pergolide metabolite is one or more kinds comprising pergolide sulfoxide, pergolide sulfone, despropyl pergolide or despropyl pergolide sulfoxide, and is preferably pergolide sulfoxide, can suppress a pergolide metabolite formation which is considered to be a main factor of side effects in the pergolide therapy, so that more remarkable reduction of the side effects being achieved.
In addition, the transdermal preparations of the invention, in which a pharmaceutically acceptable salt is one or more kinds comprising hydrochloride, sulfate, mesylate, citrate, fumarate, tartarate, maleate or acetate, can widen choices of pergolide or the like used in the pergolide therapy because wider range of salts of pergolide can be used. Among the above pharmaceutically acceptable salts mesylate is particularly preferable.
Further, the transdermal preparations of the invention those, in which the ratio (A/B) of the maximum plasma level (A) of pergolide and/or a pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2, side effects due to pergolide or the like per se can be reduced and side effects due to pergolide sulfoxide can more surely be reduced, so that more effective pergolide therapy can be carried out.

Furthermore, the transdermal preparations of the invention, in which (meth) acrylic acid copolymer is contained in an adhesive layer enables easier control of a plasma AUC ratio of pergolide or the like to at least one pergolide metabolite, more effective control of side effects in the pergolide therapy being achieved. Those further having the acrylic polymer except (meth) acrylic acid copolymer in the adhesive layer can enable more effective control of the side effects.

Further, by a transdermal preparation of the invention containing pergolide and/or a pharmaceutically acceptable salt thereof, wherein the ratio (A/B) of the maximum plasma level (A) of pergolide and/or a pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2, side effects due to pergolide or the like themselves can be reduced and/or side effects due to pergolide sulfoxide can exactly be reduced, and therefore, the pergolide therapy can be carried out effectively.
In addition, the transdermal preparations of the invention, which are adhesive patches enable extremely simple and clean administration of pergolide or the like.

### [Best Embodiment for Carrying out the Invention]

In the invention, "AUC" is abbreviation of "Area under the curve", meaning the area enclosed by a graph and X axis, which graph is obtained from each plotted point on a coordinate plane for a time course plasma level. Calculation method therefor is described, for example, in Milo Gibaldi & Donald Perrier, PharmacoKinetics, 2nd ed (1982). AUC may vary from one person to another mean value thereof maybe deviating, there is reproducibility for a general tendency and a correlation.
In the invention, "pergolide therapy" means a preventive or therapeutic treatments using pergolide or the like.
In the invention, "side effects" means all harmful actions related to use of a drug for a subject.
In the invention, "reducing side effects" means relief of frequency and/or degree of one or more kinds of side effects related to use of a drug for a subject or a subject group.
In the invention, "a pharmaceutically acceptable salt"means a salt of a drug, which exerts a desired efficacy by being administering to a subject.
In the invention, "plasma level in the next administration" means a plasma level of a drug at the next administration after one administration in repetitive administration of a drug to a subject. Therefore, "plasma level in the next administration" naturally encompasses a plasma level of the second administration.

One of the transdermal preparation of the invention is characterized in that as described above a plasma AUC of pergolide or the like to at least one pergolide metabolite is made 1:0.5 to 1:5. In view of reducing side effects due to the metabolite, the plasma AUC is preferably 1:0.5 to 1:3.5, in particular preferably 1:0.5 to 1:2.
In addition, another transdermal preparation of the invention is, with regard to the change of a drug plasma level in a repetitive administeration, a ratio (A/B) of the maximum plasma level (A) of pergolide to the plasma level (B) in the next administration and/or a ratio (A'/B') of the maximum plasma level (A') of pergolide sulfoxide to the plasma level (B') in the next administration have for the first time successfully been less than 2. In said transdermal preparation of the invention, in view of reducing side effects due to pergolide itself, (A/B) and/or (A' /B') are less than 2, preferably less than 1.5.

Further, a dosage form of the transdermal preparation of the invention is not limited as long as pergolide or the like are transdermally absorbed, so that those such as an ointment or a cream are included, an adhesive patch being preferable in view of simplicity and cleanliness in administration. Furthermore, in the case of the adhesive patch, a non-aqueous transdermal preparation is particularly preferable since pergolide and/or a pharmaceutically acceptable salt thereof being contained in a non-aqueous adhesive layer is provided with extremely favorable skin permeability with low skin irritation, in the transdermal preparation.

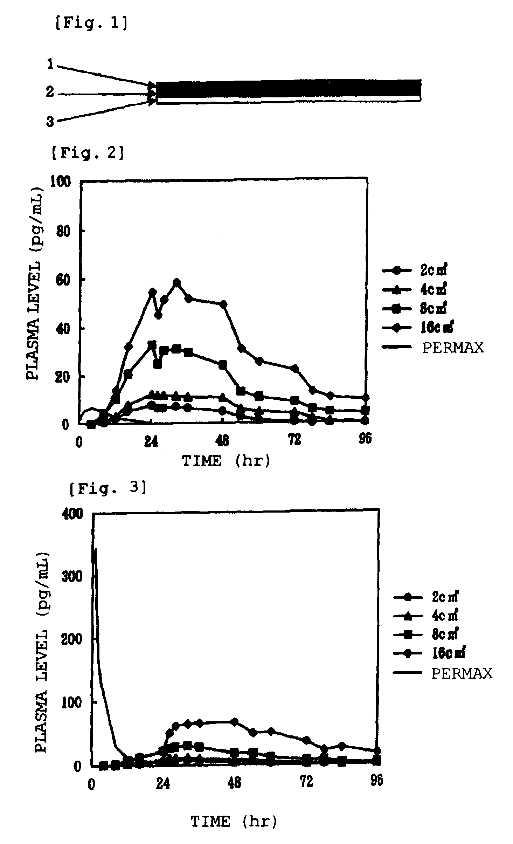

In the following, the transdermal preparation of the invention is illustrated in more detail.
The invention is a pergolide-containing transdermal preparation. Although a dosage form of the transdermal preparation of the invention is not particularly limited, the form of an adhesive patch is, for example, as shown in Fig. 1. In the following, the composition and form of the adhesive layer in the transdermal preparation of the invention are explained in detail.

A pharmacologically active ingredient in the transdermal preparation of the invention is pergolide and/or a pharmaceutically acceptable salt thereof: Pergolide or the like. The pharmaceutically acceptable salt is not particularly limited, it being possible said salt is an inorganic salt or an organic salt, while pergolide mesylate, which is a representative salt, is particularly preferable.

As for the transdermal preparation of the invention, pergolide or the like are preferably blended in an amount of 0.5-50 mass%. It becomes easy to obtain a sufficient drug permeation amount as a transdermal preparation by not less than 0. 5 mass%, and it becomes possible to keep more preferably a physical property of the preparation by not more than 50 mass%.
Further, at least one pergolide metabolite is preferably one or more kinds comprising pergolide sulfoxide, pergolide sulfone, despropyl pergolide or despropyl pergolide sulfoxide, in particular preferably pergolide sulfoxide.

In a case that the dosage form of the invention is an adhesive patch, as a base for the adhesive layer, an acrylic polymer and/or a rubber polymer are preferably used.
As the acrylic polymer there is no particular restriction as long as it is copolymer containing at least one of (meth)acrylic acid derivatives represented by 2-ethylhexyl acrylate, methyl acrylate, butyl acrylate, hydroxyethyl acrylate, 2-ethylhexyl (meth)acrylate and the like. Examples thereof are as follows: Adhesive agents described in Drug Additives Encyclopedia 2000 (edited by Japan Pharmaceutical Additive Association) as adhesive agents such as, for example, acrylic acid·octyl acrylate copolymer, 2-ethylhexyl acrylate·vinylpyrrolidone copolymer, acrylate·vinyl acetate copolymer, 2-ethylhexyl acrylate·2-ethylhexyl (meth)acrylate·dodecyl (meth)acrylate copolymer, methyl acrylate·2-ethylhexyl acrylate copolymer resin emulsion, and acrylic polymer contained in acryl resin alkanolamine liquid; DURO-TAK acryl adhesive series (manufactured by National Starch & Chemical), Eudragit series (Higuchi Shokai Co., Ltd.) and the like. (Meth)acrylic acid copolymer such as the above Eudragit series is preferable because of more effective reducing side effects. Those further containing in addition to (meth)acrylic acid copolymer further acrylic polymer are more preferable.

As the rubber polymer, styrene-isoprene-styrene block copolymer (hereinafter abbreviated as SIS), isoprene rubber, polyisobutylene (hereinafter abbreviated as PIB), styrene-butadiene-styrene block copolymer (hereinafter abbreviated as SBS), styrene-butadiene rubber (hereinafter abbreviated as SBR), polysiloxane and the like are included. Among them SIS and PIB are preferable, and SIS is in particular preferable. Such hydrophobic polymers may be used as a mixture of two or more kinds, and considering formation of an adhesive layer and sufficient permeability, the blending amount of the polymer based on the weight of the total composition is preferably 5-90 mass%, more preferably 10-70 mass%, in particular preferably 10-50 mass%.
Those further containing acrylic polymer in addition to (meth)acrylic acid copolymer is preferable.

A plasticizer may be contained in the adhesive layer of the preparations of the invention. Usable plasticizers include petroleum oils (e.g., paraffin type process oil, naphthene type process oil, aromatic type process oil and the like), squalane, squalene, vegetable oils (e.g., olive oil, camellia oil, castor oil, tall oil, peanut oil), silicone oil, dibasic acid esters (e.g., dibutyl phthalate, dioctyl phthalate and the like), liquefied rubber (e.g., polybutene, liquefied isoprene rubber), liquefied fatty acid esters (isopropyl myristate, hexyl laurate, diethyl sebacate, diisopropyl sebacate), diethylene glycol, polyethylene glycol, glycol salicylate, propylene glycol, dipropylene glycol, triacetin, triethyl citrate, crotamiton and the like. In particular, liquid paraffin, liquefied polybutene, isopropyl myristate, diethyl sebacate and hexyl laurate are preferable.

These constituents may be used with two or more kinds mixed, and considering sufficient permeability and maintenance of a sufficient cohesive force as an adhesive preparation, the blending amount of such a plasticizer based on the total composition in the adhesive layer may be 10-70 mass% in total, preferably 10-60 mass%, more preferably 10-50 mass%.

In the case of the adhesive force is not sufficient with the adhesive patch according to the invention, a tackifying resin is desirably contained in the adhesive layerUsable tackifying resins include rosin derivatives (e.g. rosin, glycerol esters of rosin, hydrogenated rosin, glycerol esters of hydrogenated rosin, pentaerythritol esters of rosin and the like), alicyclic saturated hydrocarbon resins (e.g. ARKON P 100, manufactured by Arakawa Chemical Industries, Ltd.), aliphatic hydrocarbon resins (e.g. Quintone B 170, manufactured by Zeon Corporation), terpene resins (e.g. Clearon P-125, manufactured by Yasuhara Chemical), maleic acid resins and the like. In particular, glycerol esters of hydrogenated rosin, alicyclic saturated hydrocarbon resins, aliphatic hydrocarbon resins and terpene resins are preferred.
Considering a sufficient adhesive force as an adhesive patch and irritation to the skin upon releasing, the blending amount of such a tackifying resin based on the total composition in the adhesive layer may be 5-70 mass%, preferably 5-60 mass%, more preferably 10-50 mass%.

An absorption enhancer may be contained in the adhesive layer for an adhesive patch according to the invention. As a usable absorption enhancer, any compound which shows an absorption enhancing effect may be used. Examples includes C₆-C₂₀ fatty acids, fatty alcohols, fatty acid esters, amides or ethers, aromatic organic acids, aromatic alcohols, aromatic fatty acid esters or ethers, which may be saturated or unsaturated and may be cyclic, straight or branched, furthermore lactic acid esters, acetic acid esters, monoterpene compounds, sesquiterpene compounds, Azone, Azone derivatives, pirotiodecane, glycerol fatty acid esters, propylene glycol fatty acid esters, sorbitan fatty acid esters (Span type), polysorbates (Tween type), polyethylene glycol fatty acid esters, polyoxyethylene hardened castor oils (HCO type), polyoxyethylene alkyl ethers, sucrose fatty acid esters, plant oils and the like.

Specifically, preferred are caprylic acid, capric acid, caproic acid, lauric acid, myristic acid, palmitic acid, stearic acid, isostearic acid, oleic acid, linoleic acid, linolenic acid, lauryl alcohol, myristyl alcohol, oleyl alcohol, isostearyl alcohol, cetyl alcohol, methy laurate, hexyl laurate, lauric diethanolamide, isopropyl myristate, myristyl myristate, octyldecyl myristate, cetylpalmitate, salicylic acid, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pirotiodecane and olive oil; particularly preferable are lauryl alcohol, isostearyl alcohol, lauric diethanolamide, glycerol monocaprylate, glycerol monocaprate, glycerol monooleate, sorbitan monolaurate, propylene glycol monolaurate, polyoxyethylene lauryl ether and pyrothiodecane.

Such absorption enhancers may be used with two or more kinds mixed, and considering sufficient permeability as the adhesive preparation and skin irritation such as rubor and edema, it may be blended preferably in 0.01-20 mass% based on the weight of the total composition of the adhesive layer, more preferably 0.05-10 mass%, in particular preferably 0.1-5 mass%.

In the invention it is desired to have an organic acid contained in the adhesive layer in the case that the form of pergolide is a pharmaceutically acceptable acid-addition salt. As organic acids used include aliphatic (mono-, di-, tri-)carboxylic acids (e.g. acetic acid, propionic acid, iso-butylic acid, caproic acid, caprylic acid, lactic acid, maleic acid, pyruvic acid, oxalic acid, succinic acid, tartaric acid and the like), aromatic carboxylic acids (e.g. phthalic acid, salicylic acid, benzoic acid, acetyl salicylic acid and the like), alkyl sulfonic acids (e.g. methane sulfonic acid, ethane sulfonic acid, propyl sulfonic acid, butane sulfonic acid, polyoxyethylene alkyl ether sulfonic acid and the like), alkyl sulfonic acid derivatives (e.g. N-2-hydroxyethyl-piperidine-N'-2-ethane sulfonic acid (hereinafter abbreviated as HEPES) and the like) and cholic acid (e.g. dehydrocholic acid and the like), while among them acetic acid, propionic acid, lactic acid and salicylic acid are preferable and acetic acid are in particular preferable. As to these organic acids, salts thereof or a mixture with a salt may also be used.
Considering sufficient permeation amount as the adhesive preparation and irritation to the skin, these organic acids may be blended preferably in the amount of 0.01-20 mass% based on the weight of the total composition of the adhesive layer, more preferably 0.1-15 mass%, in particular preferably 0.1-10 mass%.

In addition, if needed, an anti-oxidant, filler, cross-linking agent, preservative and UV absorber can be used. As anti-oxidants, tocopherol and its ester derivatives, ascorbic acid, ascorbic acid-stearic acid ester, nordihydroguaretic acid, dibutyl hydroxy toluene (BHT), butyl hydroxy anisole and the like are desirable. As fillers, calcium carbonate, magnesium carbonate, silicate (e.g. aluminum silicate, magnesium silicate and the like), silicic acid, barium sulfate, calcium sulfate, calcium zincate, zinc oxide, titanic oxide and the like are desirable. As cross-linking agents, thermosetting resins such as amino resins, phenol resins, epoxy resins, alkyd resins and unsaturated polyesters, isocyanate compounds, block isocyanate compounds, organic type cross-linking agents, and inorganic type cross-linking agents such as metals or metal compounds, are desirable. As preservatives, ethyl p-hydroxy benzoate, propyl p-hydroxy benzoate, butyl p-hydroxy benzoate and the like are desirable. As UV absorbers, p-amino benzoic acid derivatives, anthranilic acid derivatives, salicylic acid derivatives, coumarin derivatives, amino acid type compounds, imidazoline derivatives, pyrimidine derivatives, dioxane derivatives and the like are desirable.

Such antioxidant, filler, cross-linking agent, preservative and UV absorber may be blended preferably with an amount of not more than 10 mass% in total based on the weight of the total composition in the adhesive layer of the adhesive preparation, more preferably not more than 5 mass% and in particular preferably not more than 2 mass%.

A drug-containing adhesive layer having the composition described above can be prepared by any method. For example, a composition of a drug-containing base is heat-melted, coated on a release liner or a backing, followed by affixing to the backing or the release liner to give the present preparations. In addition, base constituents containing a drug are dissolved in solvent such as toluene, hexane or ethyl acetate, spreadon the release liner or the backing layer, dried to remove solvent, followed by affixing to the backing or the release paper to give the present preparations.

The backing layer for an adhesive patch according to the invention is not particularly limited as long as it is appropriate for supporting the adhesive layer, although a stretch or non-stretch material may be used. For example, fabric, no-woven fabric, polyurethane, polyester, polyvinyl acetate, polyvinylidene chloride, polyethylene, polyethylene terephthalate, aluminum sheet and the like, or composite materials thereof may be used.

### [Example]

In the following, the invention is explained in more detail by the examples. The invention, however, is not limited to these examples, and various modifications may be made without departing from the technical spirit of the invention. Further, in the examples, all % mean % by mass.

### (Example 1)

| | |
|---|---|
| SIS | 10.0% |
| Acryl adhesive agent (DURO-TAK87-2194) | 8.0% |
| Acrylic polymer (Eudragit EPO) | 10.0% |
| Alicyclic saturated hydrocarbon resin (ARKON P 100) | 35.0% |
| Liquid paraffin | 15.0% |
| Acetic acid | 6.0% |
| Sodium acetate | 2.0% |
| Pergolide mesylate | 9.0% |
| Sorbitan monolaurate | 2.0% |
| Isostearyl alcohol | 3.0% |
| Total amount | 100.0% |

Pergolide mesylate, acetic acid, sodium acetate, sorbitan monolaurate, isosteryl alchol and liquid paraffin were beforehand put in a mortar, and mixed thoroughly, followed by mix with remaining constituents dissolved in ethyl acetate and heptane. After the mixture was spread on a release liner, solvent was removed by drying, followed by affixing to a PET film backing to give the transdermal preparation of the invention.

### (Test example)

### (1) Skin permeation test in hairless mice

A back part skin of a hairless mouse was stripped, and the dermal side was placed to a receptor cell side and mounted on a flow-through cell (5 cm²) in which warm water of 37°C was circulated around the outer part. The preparation obtained in Example 1 was applied on the stratum corneum side, and samplings were carried out at every two hour for 24 hours at a rate of 5 ml/hour (hr) using the physiological saline in the receptor layer. For the receptor solution obtained at each hour, the flow amount was accurately measured, and the drug level was measured by a high-performance liquid chromatography. Based on the flow amount and the measured value of the drug level, the permeation rate per hour was calculated, and the maximum skin permeation rate for each example was obtained. The result is shown in Table 1.

### (2) Skin permeation test in human skin sample

Measurement was done in a similar way to the skin permeation test in hairless mice. The result of the maximum skin permeation rate for the preparation obtained in Example 1 is shown in Table 1.

### (3) Primary skin irritation test in rabbits

The primary skin irritation of the preparation obtained in Example 1 was tested according to draize method. The PII value obtained in each preparation is shown in Table 1.

### (4) Physical test of preparations

The adhesive force of the preparation obtained in Example 1 was measured by a prove tack tester and a peel measuring instrument, and the cohesive force by using a creep measuring instrument. As the result, one having no problem in the physical properties was evaluated as ○ and one having a problem was evaluated as ×. The result is shown in Table 1.

**[Table 1]**

| Maximum skin permeation rate (µg/cm²/hr) | | | | |
|---|---|---|---|---|
| | Hairless mice | Human subjects | PII value | Physical property of preparation |
| Example 1 | 9.03 | 1.19 | 0.83 | ○ |

### (5) Pharmacokinetic test in human single administration

The pharmacokinetic parameter of unchanged substance in plasma in case of a single administration of the preparation obtained in Example 1 to healthy adults (n=8) and the pharmacokinetic parameter of pergolide sulfoxide, main metabolite, are shown in Table 2 and Table 3 respectively. In addition, the pharmacokinetic parameter of Permax which is an oral preparation of pergolide is also shown simultaneously for comparison.
The plasma levels which are the basis of these results are shown in Fig. 2 and Fig. 3 respectively.
In addition, the degree of side effects in each treatment part is shown in Table 4.

**[Table 2]**

| | Administration amount | Tmax (hr) | Cmax (pg/mL) | AUC_{0-∞} (pg·hr/mL) |
|---|---|---|---|---|
| Permax Tablet | 50µpg | 3.4 | 6.7 | 98.0 |
| Example 1 | 1.35mg(2cm²) | 30.5 | 8.1 | 359.0 |
| | 2.7mg(4cm²) | 32.8 | 14.5 | 633.8 |
| | 5.4mg(8cm²) | 31.0 | 34.6 | 1578.3 |
| | 10.8mg(16cm²) | 35.0 | 69.2 | 3089.9 |

**[Table 3]**

| | Administration amount | Tmax (hr) | Cmax (pg/mL) | AUC_{0-∞} (pg·hr/mL) |
|---|---|---|---|---|
| Permax Tablet | 50µg | 0.7 | 417.0 | 1384.2 |
| Example 1 | 1.35mg (2cm²) | 28.5 | 8.3 | 369.8 |
| | 2.7mg (4cm²) | 32.8 | 11.9 | 578.3 |
| | 5.4mg (8cm²) | 32.0 | 30.0 | 1312.6 |
| | 10.8mg (16cm²) | 37.8 | 74.1 | 3478.5 |

**[Table 4]**

| Permax Tablet | | | Example 1 | | |
|---|---|---|---|---|---|
| | 50µg | 2cm² Preparation | 4cm² Preparation | 8cm² Preparation | 16cm² Preparation |
| Sleepiness | 4 | 1 | 0 | 0 | 0 |
| Vomiting | 1 | 1 | 0 | 0 | 0 |
| Headache | 1 | 0 | 0 | 0 | 0 |
| Total | 6 | 2 | 0 | 0 | 0 |

Based on Tables 2 and 3, the AUC ratios of pergolide mesylate to its main metabolite, pergolide sulfoxide, in Permax tablet and Example 1 (administration amounts, 1.35mg, 2.7mg, 5.4mg and 10.8mg; corresponding to 2cm², 4cm², 8cm² and 16cm² respectively) became 1:14.1, 1:1.03, 1:0.91, 1:0.83 and 1:0.89 respectively. Namely, in the transdermal preparation of the invention, the AUC ratios of pergolide mesylate to its metabolite were 1/17 to 1/14 compared with Permax tablet, being extremely small. Further, the side effects in this case (sleepiness, vomiting and headache) were significantly small in case of administering the transdermal preparation of the invention compared with administering Permax tablet (Table 4). In particular, in the administration parts of 2.7mg, 5.4mg and 10.8mg, in which the above AUC ratios were not more than 1:1.0, the above side effects were not observed at all.

### (6) Simulation for repetitive administeration

Among each drug preparation used in (5), the plasma levels of pergolide and pergolide sulfoxide in case of repetitively administering 8cm² preparation and 16cm² preparation related to the example of the invention and Permax tablet of oral preparation at 24 hr interval were obtained by the following simulation. Further, as to pergolide sulfoxide, the simulation was carried out only for 16cm² preparation.
In the transdermal preparation of the invention, the plasma level measured in a single administration was successively added every 24 hr, and a plasma level change when repetitively administering was calculated. The calculation was carried out at 2 hr interval, while as to a point in which there is no measured value of a plasma level in the single administration, it was obtained by interpolation of values before and after the point. As to the values after 96 hr, an extrapolated value was used so that the plasma level became substantially 0pg/ml.
As to Permax tablet, the simulation was carried out in a similar manner to the above transdermal preparation of the invention in accordance to a standard administration method.

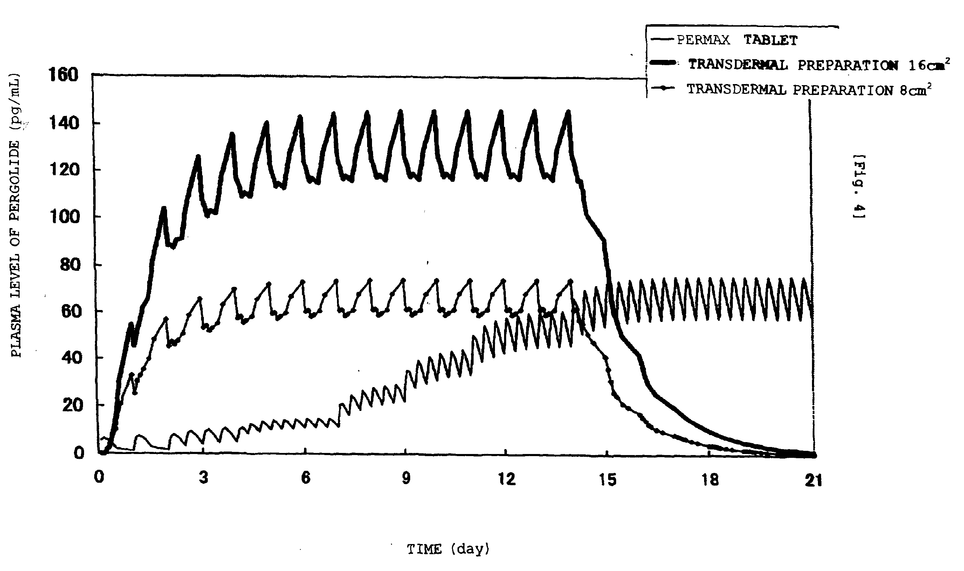

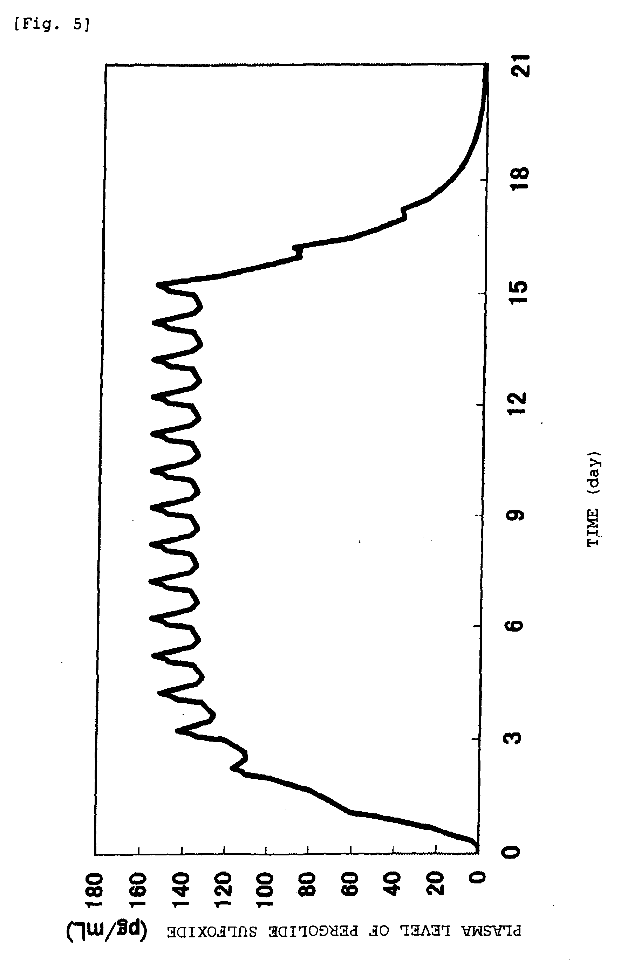

As the results, the ratio (A/B) of the maximum plasma level (A) of pergolide in the first administration to the plasma level (B) at the time of the second administration and the ratio (A'/B') of the maximum plasma level (A') of pergolide sulfoxide in the first administration to the plasma level (B') at the time of the second administration became 1.0 parallel to the fact that Tmax exceeded 30 hr (Fig. 4 and Fig. 5).
In the mean time, in Permax the maximum plasma level (A) of pergolide after the first administration was 6.70pg/ml, and the plasma level (B) at the time of the second administration, that is, after 24 hr from the first administration was 1.35pg/ml. Therefore, in Permax A/B was 4.96 (6.70/1.35) (Fig. 4).

Based on the above results, it was confirmed that the pergolide-containing transdermal preparation of the invention was excellent in a drug permeation rate and was sufficiently good for a practical use concerning a skin irritation and a physical property of the preparation. In addition, in the transdermal preparation of the invention it also became apparent that side effects accompanied with a metabolite were remarkably reduced because the formation amount of at least one metabolite was extremely low compared with the conventional oral preparation.
Further, in the transdermal preparation of the invention it was confirmed that side effects of pergolide itself and its main metabolite could remarkably be reduced because the ratio (A/B) of the maximum plasma level (A) of pergolide in the first administration to the plasma level (B) at the time of the second administration and the ratio (A'/B') of the maximum plasma level (A') of pergolide sulfoxide in the first administration to the plasma level (B') at the time of the second administration can be led to less than 2.

### [Industrial Applicability]

By using a pergolide-containing transdermal preparation of the invention, it is possible to let pergolide and/or a pharmaceutically acceptable salt thereof be absorbed effectively in circulating blood via the skin, and the preparation can simply be administered. In addition, by the pergolide-containing transdermal preparation of the invention, it is possible to reduce the side effects by suppressing the formation of at least one metabolite. Further, the pergolide-containing transdermal preparation of the invention has favorable stickiness to the skin and is useful as a preparation for external use which makes a trasdermal application of pergolide in a parkinsonism therapy an object.
Therefore, the pergolide-containing transdermal preparation of the invention greatly contributes to the development of pharmaceutical industry as well as related industries.

### [Brief Description of Drawing]

Figure 1 shows one embodiment of a pergolide-containing transdermal preparation of the invention.
Figire 2 shows a age change of the plasma level of unchanged substance in case of a single administration of a pergolide-containing transdermal preparation of the invention.
Figure 3 shows a age change of the plasma level of the main metabolite (pergolide sulfoxide) in case of a single administration of a pergolide-containing transdermal preparation of the invention.
Figure 4 shows a simulation result of the age change of the plasma level of pergolide in case of a repetitive administration of a pergolide-containing transdermal preparation of the invention.
Figure 5 shows a simulation result of the age change of the plasma level of pergolide sulfoxide in case of a repetitive administration of a pergolide-containing transdermal preparation of the invention.

### Description of Symbols]

1: Backing layer
2: Adhesive layer containing a drug
3: Liner layer

## Claims

1. A transdermal preparation containing pergolide and/or a pharmaceutically acceptable salt thereof, wherein said preparation is capable of achieving a plasma AUC ratio of pergolide or the pharmaceutically acceptable salt thereof to at least one metabolite thereof of 1:0.5 to 1:5.

2. The transdermal preparation according to claim 1, wherein the plasma AUC ratio of pergolide and/or a pharmaceutically acceptable salt thereof to at least one metabolite thereof is 1:0.5 to 1:3.5.

3. The transdermal preparation according to claim 2, wherein the plasma AUC ratio of pergolide and/or a pharmaceutically acceptable salt thereof to at least one metabolite thereof is 1:0.5 to 1:2.

4. The transdermal preparation according to any one of claims 1 to 3, wherein the metabolite is one or more kinds comprising pergolide sulfoxide, pergolide sulfone, despropyl pergolide or despropyl pergolide sulfoxide.

5. The transdermal preparation according to claim 4, wherein the metabolite is pergolide sulfoxide.

6. The transdermal preparation according to any one of claims 1 to 5, wherein the pharmaceutically acceptable salt is one or more kinds comprising hydrochloride, sulfate, mesylate, citrate, fumarate, tartrate, maleate or acetate.

7. The transdermal preparation according to claim 6, wherein the pharmaceutically acceptable salt is mesylate.

8. The transdermal preparation according to any one of claims 1 to 7, wherein the ratio (A/B) of the maximum plasma level (A) of pergolide and/or the pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2.

9. The transdermal preparation according to any one of claims 1 to 8, wherein (meth)acrylic acid copolymer is contained in an adhesive layer.

10. The transdermal preparation according to claim 9, wherein the acrylic polymer except (meth)acrylic acid copolymer is further contained in an adhesive layer.

11. A transdermal preparation containing pergolide and/or the pharmaceutically acceptable salt thereof, wherein the ratio (A/B) of the maximum plasma level (A) of pergolide and/or the pharmaceutically acceptable salt thereof to the plasma level (B) thereof in the next administration and/or the ratio (A'/B') of the maximum plasma level (A') of pergolide sulfoxide to the plasma level (B') of pergolide sulfoxide in the next administration is less than 2.

12. The transdermal preparation according to any one of claims 1 to 11, wherein said preparation is an adhesive patch.
